# EUROPEAN PATENT APPLICATION

(11) **EP 1 550 460 A1**
(43) Date of publication of application: **06.07.2005**
(21) Application number: 02759005.8
(22) Date of filing: 02.08.2002
(51) Int. Cl.: A61K 39/395, A61P 37/00

(54) **METHOD FOR CORRECTING IMMUNE RESPONSES AND MEDICINAL AGENT**

(71) Applicant: Epshtein, Oleg Iliich, Moscow, 103064 (RU); Goldberg, Evgeny Danilovich, Tomsk, 634029 (RU); Dygay, Alexandr Mikhailovich, Tomsk, 634050 (RU)
(72) Inventor: EPSHTEIN, Oleg Iliich, Moscow, 103064 (RU)
(74) Representative: BOVARD AG - Patentanwälte
(86) International application number: PCT/RU2002/000367
(87) International publication number: WO 2004/012766

(57) **Abstract**

The principle of the present invention is the use of an activated form of ultra-low doses of monoclonal, polyclonal, or natural antibodies to cytokines implicated in the course of inflammation, including autoimmune inflammation, the activated form being prepared by multiple consecutive dilutions and exposure to external factors, preferably according to the homeopathic technology. It is possible to use a mixture of activated forms of antibodies to various cytokines involved in the course of the inflammatory process. In addition, employed as a medicament for correcting pathologic immune reactions based on antibodies to the tumor necrosis factor alpha (TNF-α) can be an activated form of antibodies to TNF-α obtained by multiple consecutive dilutions and exposure to external factors; activation preferably being performed in accordance with the homeopathic technology, and the forms preferably being applied in a mixture of various, predominantly centesimal, homeopathic dilutions.

## Description

### Field of the invention

The preset invention relates to the field of medicine and can be used for correction of pathologic immune reactions associated with inflammatory, including autoimmune, processes.

### Background of the invention

It is a well-known practice to treat autoimmune diseases by introducing antibodies to various cytokines regulating the course of inflammatory processes, including autoimmune inflammation. In these cases antibodies to the tumor necrosis factor alpha (TNF-α) are employed as therapeutics (see Maini RN, Taylor PC. Anti-Cytokine Therapy for Rheumatoid Arthritis, Annual Reviews in Medicine, 2000;51:207-229).

However, the manufacturing of such therapeutics is complicated; the use of whole heterologous antibodies is not possible (thus chimeric antibodies are used). The medications are administered in relatively high doses (up to 5 mg/kg body weight). The therapeutic effect of such preparations is based on cytokine binding (inactivating).

### Description of the invention

The present invention is directed at obtaining a preparation with immunotropic activity, mainly for the treatment of inflammatory, including autoimmune, diseases, the preparation ensuring the anti-inflammatory effect by modification rather than by binding (inactivation) of a cytokine. The new medicament provides an effect synergic with that of the cytokine.

The formulated objective is attained by the use of an activated form of ultra-low doses of monoclonal, polyclonal or natural antibodies to cytokines regulating the course of inflammatory processes, including autoimmune inflammations, for the correction of pathologic immune reactions; this form is prepared by multiple consecutive dilutions and by exposure to external factors, preferably according to homeopathic technology.

A mixture of activated forms of antibodies to various cytokines regulating the course of inflammatory processes can be used.

In addition, the medicament based on antibodies to the tumor necrosis factor alpha (TNF-α) used for the correction of pathologic immune reactions may contain an activated form of antibodies to TNF-α obtained by multiple consecutive dilutions and exposure to external factors, preferably by homeopathic technology.

The antibodies are obtained with the use of human or heterologous tumor necrosis factor alpha, including a recombinant factor.

It is preferred to employ a mixture of various, preferably centesimal, homeopathic dilutions.

The medicament obtained in accordance with the present invention is a new pharmaceutical characterized by a prominent specific pharmacological activity. The anti-inflammatory effect, unlike that of the antibodies in physiologic and/or therapeutic doses, is due not to TNF-α blockage but to its modification (effect synergic to that of TNF-α itself).

The existence of the therapeutic effect of ultra-low doses of antibodies activated by homeopathic technology, as well as the unidirectional character of the action with the original molecule do not follow from the state-of-the-art knowledge and have been discovered by the inventor.

### Embodiments of the invention

The new medicament is preferably prepared in the following manner.

Recombinant human tumor necrosis factor alpha expressed in *Escherichia coli* is purified by electrophoresis to at least 97 % of the active principle and used as an immunogen for immunization of rabbits. The resultant immune polyclonal antibodies are purified by affinity chromatography with Protein A.

The method of preparation of polyclonal antibodies is described, for instance, in the book G. Frimel, Ed., Immunological Methods (in Russian), Moscow, Meditsina, 1987, pp. 9-33.

The isolated antibodies to the recombinant human tumor necrosis factor alpha are subjected to consecutive multiple dilutions and to an external mechanical factor until ultra-low or low doses are obtained, for example, by the homeopathic potentisation technology (see W. Schwabe, Homöopathisches Arzneibuch, Stuttgart, 1978). This procedure gives rise to a uniform decrease in the concentration through consecutive dilution of 1 volumetric part of the initial matter (antibodies) in 9 volumetric parts (for decimal dilution, D) or in 99 volumetric parts (for centesimal dilution, C) of a neutral solvent with multiple vertical shaking of each solution; the advantages of preferably various containers for each subsequent dilution are used. Finally, this procedure gives the required dose (potency).

The external treatment in the course of concentration reduction can also be executed by exposure to ultrasonic, electromagnetic, or other physical factors.

The resultant medicines are used mostly in the dosage forms and dilutions adopted in the homeopathic practice: as alcoholic and aqueous solutions or as tablets (granules) prepared by impregnating the carrier contained in the dosage form by the potentised solution to saturation; also, the potentised solution can be added directly to a liquid dosage form.

### Example 1

In studies of the anti-inflammatory action of activated forms of ultra-low doses of antibodies to TNF-α we induced immune inflammation in male rats using Freund's complete adjuvant. The phlogogen was injected in a single dose into the cushion of the paw (under the plantar aponeurosis). Polyclonal rabbit antibodies to the rat TNF-α in the form of a mixture of homeopathic dilutions C12 + C30 + C200 were administered *per os* through a pump daily (0.5 ml per rat) beginning from the day before adjuvant inoculation within a period of 15 days (during the whole course of the inflammatory process development). As a criterion of the process we used the intensity of hyperemia and edema of the right and left extremities measured oncometrically every 2 days.

An analysis of the data on the development of the edema of the extremity revealed that the preparation decreased efficiently the intensity of the edema (by 50-80 % as compared with the control) in the course of the secondary inflammatory reaction appearing on the 10^{th} day.

### Example 2

In studies of the analgetic action of activated forms of ultra-low doses of antibodies to anti-inflammatory cytokines in male rats, we induced immune inflammation using Freund's complete adjuvant. The phlogogen was injected in a single dose into the cushion of the paw (under the plantar aponeurosis). A mixture of polyclonal rabbit antibodies to the rat TNF-α and the rat Interleukin 8 in the form of a solution with homeopathic dilution C30 was administered *per os* through a pump every day (0.5 ml per rat) beginning from the day before adjuvant inoculation within a period of 15 days (during the whole course of the inflammatory process development).

The analgetic action of the preparation on pain threshold of the inflamed tissues was studied in hot plate test. The animals were placed on a hot plate, and the latency of the stay on the plate until licking the inflamed paw was registered. The data were recorded every 3 hours and on the 1^{st} and 3^{rd} day after phlogogen injection.

The administration of a mixture of antibodies to TNF-α and Interleukin 8 increased the time of stay on the hot plate on the 1^{st} and 3^{rd} day by a factor of 1.7 and 3.0, respectively, as against the control (distilled water).

### Example 3

Patient K. (male), aged 57, had been suffering from rheumatoid arthritis (RA, Class III by the functional classification of the American College of Rheumatologists) for 5 years; he was hospitalized because of the disease exacerbation. He complained of fever, increased morning stiffness, pain and edema in the affected joints. Clinical findings: temperature 37.5 °C, pronounced hyperemia and defiguration of the wrist, ankle, and proximal interphalangeal joints, pain on palpation. Blood: ESR 35 mm/h, rheumatoid factor ++. Because of poor tolerance of non-steroid anti-inflammatory drugs, monoclonal antibodies to recombinant human tumor necrosis factor alpha in a mixture of homeopathic dilutions C50, C200, and C1000 (1 tablet, 3 times a day) was prescribed. Three days after the beginning of the treatment the patient noted a substantial attenuation of the pain syndrome, the body temperature decreased to the norm. By the 7^{th} day of the treatment the morning stiffness noted before hospitalization remained. The patient was discharged on the 14^{th} day with clinical-laboratory remission. Recommendation: preventive administration of the preparation (1 tablet every other day). Two months after the discharge the patient's Class III of the RA functional classification was changed to Class II.

### Example 4

Patient U. (female), aged 67, had been suffering from right-side coxarthrosis for 10 years. She applied to the physician because of the exacerbation of the night pain, the pain on motion, and lessening of the joint's mobility. Prescription: rabbit antibodies to the recombinant human tumor necrosis factor alpha (TNF-α) in a mixture of homeopathic C12, C30, and C200 dilutions in an aqueous solution: 5 ml, 3 times a day. Three days after the beginning of the treatment the patient noted the disappearance of the night pain and pain on motion. On the 7^{th} day of the preparation intake the mobility in the right hip joint returned to the initial level (before the exacerbation). As a result of the 2 months' course of the intake of antibodies to TNF-α, X-ray examination revealed the regression of the roentgenologic signs of coxarthrosis. This process was accompanied by the growth of the amplitude of passive and active motions in the joint. During the whole period of treatment the patient marked the absence of joint pains.

## Claims

1. A method of correcting pathologic immune reactions by administering antibodies to cytokines **characterized by** the use of activated form of ultra-low doses of monoclonal, polyclonal, or natural antibodies to cytokines involved in the course of inflammation, including autoimmune inflammation, the activated form being prepared by multiple consecutive dilutions and exposure to external factors, preferably following the homeopathic technology.

2. A method of treatment according to Claim 1 **characterized by** the use of a mixture of activated forms of antibodies to various cytokines involved in the course of the inflammatory process.

3. A medicament for correction of pathologic immune reactions based on antibodies to tumor necrosis factor alpha (TNF-α) **characterized by** the presence of an activated form of antibodies to TNF-α obtained by multiple consecutive dilutions and exposure to external factors, preferably following the homeopathic technology.

4. A medicament according to Claim 3 **characterized by** obtaining antibodies with the use of human or heterologous tumor necrosis factor alpha (TNF-α), including a recombinant factor.

5. A medicament according to Claim 3 or 4 **characterized by** containing a mixture of various, mostly centesimal, homeopathic dilutions.
